# EUROPEAN PATENT SPECIFICATION

(11) **EP 4 279 478 B1**
(45) Date of publication and mention of the grant of the patent: **09.10.2024**
(21) Application number: 21923691.6
(22) Date of filing: 03.02.2021
(51) Int. Cl.: C07C 51/09, C07C 59/06

(54) **METHOD FOR PREPARING GLYCOLIC ACID THROUGH HYDROLYSIS OF ALKOXYACETATE**
VERFAHREN ZUR HERSTELLUNG VON GLYCOLSÄURE DURCH HYDROLYSE VON ALKOXYACETAT
PROCÉDÉ DE PRÉPARATION D'ACIDE GLYCOLIQUE PAR HYDROLYSE D'ALCOXYACÉTATE

(43) Date of publication of application: 22.11.2023
(73) Proprietor: Dalian Institute of Chemical Physics, Chinese Academy of Sciences, Dalian, Liaoning 116023 (CN)
(72) Inventor: NI, Youming, Dalian, Liaoning 116023 (CN); ZHU, Wenliang, Dalian, Liaoning 116023 (CN); LIU, Zhongmin, Dalian, Liaoning 116023 (CN)
(74) Representative: Bayramoglu et al.
(86) International application number: PCT/CN2021/075027
(87) International publication number: WO 2022/165663

(56) References cited:
- CN-A- 1 180 067
- CN-A- 106 554 250
- CN-A- 107 445 825
- JP-A- 2003 300 926
- FÖLSING AUGUST: "Ueber die Einwirkung von Bromwasserstoff auf die Aetherester der Oxysäuren", BERICHTE DER DEUTSCHEN CHEMISCHEN GESELLSCHAFT, vol. 17, no. 1, 1 January 1884 (1884-01-01), DE, pages 484 - 486, XP093131026, ISSN: 0365-9496, Retrieved from the Internet <URL:https://onlinelibrary.wiley.com/doi/pdf/10.1002/cber.188401701135> DOI: 10.1002/cber.188401701135

## Description

### TECHNICAL FIELD

The present application relates to a method for preparing glycolic acid through hydrolysis of alkoxyacetate, and belongs to the field of preparation of chemical products.

### BACKGROUND

Glycolic acid, also known as hydroxyacetic acid, is the simplest α-hydroxycarboxylic acid compound. Because the molecular structure of glycolic acid includes both hydroxyl and carboxyl, glycolic acid can undergo self-polymerization to produce polyglycolic acid (PGA). PGA has not only excellent biocompatibility, but also safe biodegradability. Therefore, PGA is widely used not only for medical surgical sutures, drug sustained-release materials, and degradable human tissue scaffolds, but also in production of common plastic products. Conventional non-degradable plastic products have caused heavy environmental pollution, and biodegradable PGA plastics are expected to solve this problem. Glycolic acid can also undergo copolymerization with monomers such as lactic acid and hydroxypropionic acid to produce a polymer material with excellent performance and extensive applications. In addition, glycolic acid is an excellent chemical cleaning agent and cosmetic raw material.

Preparation methods of glycolic acid mainly include a chloroacetic acid hydrolysis method, a formaldehyde carbonylation method, and an oxalate hydrogenation/hydrolysis method. In the chloroacetic acid hydrolysis method, the preparation process of the raw material chloroacetic acid causes heavy pollution, and the hydrolysis process of chloroacetic acid leads to a large amount of waste salt, heavy pollution, and poor quality product. Thus, the chloroacetic acid hydrolysis method has been basically eliminated. The formaldehyde carbonylation method involves cheap and easily-available raw materials, but needs to be implemented under conditions such as a high temperature, a high pressure, a strong liquid acid, and an organic solvent. In addition, in the formaldehyde carbonylation method, the device is easily corroded, and the product purification is difficult, resulting in a high industrial production cost. In the oxalate hydrogenation/hydrolysis method, an oxalate is partially hydrogenated to produce methyl glycolate, and then methyl glycolate is hydrolyzed to produce glycolic acid. However, the catalyst for partial hydrogenation of the oxalate is not mature and has low conversion efficiency and poor stability; and the oxalate production process is cumbersome and costly. These problems seriously restrict the development of the oxalate hydrogenation/hydrolysis method.

The publication "Ueber die Einwirkung von Bromwasserstoff auf die Aetherester der Oxysäuren",BERICHTE DER DEUTSCHEN CHEMISCHEN GESELLSCHAFT, vol. 17, no. 1, 1 January 1884 (1884-01-01), pages 484-486, by A. Fölsing discloses the hydrolysis of the methyl methoxyacetate to glycolic using gaseous HBr.

### SUMMARY

According to the problems faced by the existing production technologies of glycolic acid (HOCH₂COOH), the present disclosure discloses a method for preparing glycolic acid through hydrolysis of alkoxyacetate. The method of the present application is particularly suitable for carbonylation of DMM produced in the coal chemical industry to produce methyl methoxyacetate and then hydrolysis to produce glycolic acid.

A method for preparing glycolic acid through hydrolysis of alkoxyacetate is provided, including: subjecting raw materials including the alkoxyacetate and water to a reaction in the presence of an acidic molecular sieve catalyst to produce the glycolic acid,
where the alkoxyacetate is at least one selected from the group consisting of compounds with a structural formula shown in formula I: 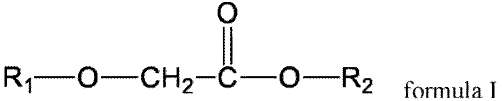
where R₁ and R₂ each are independently any one selected from the group consisting of C₁-C₅ alkyl groups.

The present application discloses a method for preparing glycolic acid through hydrolysis of alkoxyacetate, where raw materials of alkoxyacetate and water are allowed to pass through a reaction zone loaded with an acidic molecular sieve catalyst, such that the raw materials undergo a reaction under specified conditions to produce glycolic acid. The hydrolysis catalyst used in the present application is a molecular sieve catalyst with long life and high hydrolysis efficiency. The glycolic acid production method in the present application can be implemented by a traditional fixed-bed reactor under an atmospheric pressure, which is very suitable for continuous production. The raw material alkoxyacetate in the present application can be prepared by an environmentally-friendly and economical acetal carbonylation method. When the raw material in the present application is methyl methoxyacetate, methanol and formaldehyde condensation to prepare dimethoxymethane (DMM), DMM carbonylation to prepare methyl methoxyacetate, and methyl methoxyacetate hydrolysis to prepare glycolic acid can be used in combination to allow efficient, environmentally-friendly, and economical conversion of methanol (a platform chemical in the coal chemical industry) into glycolic acid.

Preferably, R₁ is any one selected from the group consisting of methyl, ethyl, propyl, and butyl; and
R₂ is any one selected from the group consisting of methyl, ethyl, propyl, and butyl.

Specifically, the alkoxyacetate is any one selected from the group consisting of methyl methoxyacetate, ethyl methoxyacetate, n-propyl methoxyacetate, n-butyl methoxyacetate, and ethyl ethoxyacetate.

Further preferably, the alkoxyacetate is methyl methoxyacetate.

In recent years, the DMM carbonylation reaction to prepare methyl methoxyacetate has received widespread attention. This reaction is based on a molecular sieve catalyst, can be conducted at a low reaction temperature, and has high atomic economy. The raw material DMM can be produced by a very mature industrialized technology with high efficiency, and thus is cheap. In the present application, the hydrolysis of ether and ester bonds of methyl methoxyacetate to prepare glycolic acid becomes an environmentally-friendly and economical production path for glycolic acid.

Optionally, the acidic molecular sieve catalyst includes an acidic molecular sieve.

Optionally, the acidic molecular sieve is at least one selected from the group consisting of an acidic MFI-structured molecular sieve, an acidic FAU-structured molecular sieve, an acidic FER-structured molecular sieve, an acidic BEA-structured molecular sieve, an acidic mordenite (MOR)-structured molecular sieve, and an acidic MWW-structured molecular sieve.

Preferably, the acidic molecular sieve is any one selected from the group consisting of an acidic MFI-structured molecular sieve and an acidic FER-structured molecular sieve.

Optionally, the acidic molecular sieve is at least one selected from the group consisting of an acidic ZSM-5 molecular sieve, an acidic Y molecular sieve, an acidic ZSM-35 molecular sieve, an acidic β molecular sieve, an acidic MOR molecular sieve, and an acidic MCM-22 molecular sieve.

Preferably, the acidic molecular sieve is at least one selected from the group consisting of a hydrogen-type ZSM-5 molecular sieve, a hydrogen-type Y molecular sieve, a hydrogen-type ZSM-35 molecular sieve, a hydrogen-type β molecular sieve, a hydrogen-type MOR molecular sieve, and a hydrogen-type MCM-22 molecular sieve.

Further preferably, the acidic molecular sieve is any one selected from the group consisting of a hydrogen-type ZSM-5 molecular sieve and a hydrogen-type ZSM-35 molecular sieve.

Optionally, a Si/Al atom ratio of the acidic molecular sieve is 3 to 500.

Specifically, an upper limit of the Si/Al atom ratio of the acidic molecular sieve is selected from the group consisting of 10, 20, 50, 100, and 500; and a lower limit of the Si/Al atom ratio of the acidic molecular sieve is selected from the group consisting of 3, 10, 20, 50, and 100.

Optionally, the acidic molecular sieve catalyst further includes a forming agent; and
the forming agent is an oxide.

Optionally, the oxide is at least one selected from the group consisting of alumina and silicon oxide.

Optionally, a content of the forming agent in the acidic molecular sieve catalyst is m, and 0 < m ≤ 50 wt%.

Specifically, an upper limit of the content of the forming agent in the acidic molecular sieve catalyst is selected from the group consisting of 10 wt%, 20 wt%, 40 wt%, and 50 wt%; and a lower limit of the content of the forming agent in the acidic molecular sieve catalyst is selected from the group consisting of 5 wt%, 10 wt%, 20 wt%, and 40 wt%.

Preferably, the content of the forming agent in the acidic molecular sieve catalyst is 15 wt% to 25 wt%.

Optionally, conditions of the reaction are as follows:
a reaction temperature is 60°C to 260°C;
a reaction pressure is 0.1 MPa to 10 MPa;
a molar ratio of the alkoxyacetate to the water is 1:20 to 20:1; and
a weight hourly space velocity (WHSV) of the alkoxyacetate is 0.1 h⁻¹ to 3 h⁻¹.

Specifically, an upper limit of the reaction temperature is selected from the group consisting of 100°C, 150°C, and 260°C; and a lower limit of the reaction temperature is selected from the group consisting of 60°C, 100°C, and 150°C.

An upper limit of the reaction pressure is selected from the group consisting of 0.3 MPa, 0.5 MPa, 1 MPa, 4 MPa, and 10 MPa; and a lower limit of the reaction pressure is selected from the group consisting of 0.1 MPa, 0.3 MPa, 0.5 MPa, 1 MPa, and 4 MPa.

An upper limit of the molar ratio of the alkoxyacetate to the water is selected from the group consisting of 1:10, 1:8, 1:4, 1:2, 1: 1, 10: 1, and 20: 1; and a lower limit of the molar ratio of the alkoxyacetate to the water is selected from the group consisting of 1:20, 1:10, 1:8, 1:4, 1:2, 1:1, and 10: 1.

An upper limit of the WHSV of the alkoxyacetate is selected from the group consisting of 0.3 h⁻¹, 1.0 h⁻¹, and 3.0 h⁻¹; and a lower limit of the WHSV of the alkoxyacetate is selected from the group consisting of 0.1 h⁻¹, 0.3 h⁻¹, and 1.0 h⁻¹.

Preferably, the conditions of the reaction are as follows:
a reaction temperature is 130°C to 260°C;
a reaction pressure is 0.1 MPa to 0.3 MPa;
a molar ratio of the alkoxyacetate to the water is 1:2 to 1:8; and
a WHSV of the alkoxyacetate is 0.3 h⁻¹ to 1 h⁻¹.

Optionally, the reaction is conducted in at least one fixed-bed reactor; and
when the reaction is conducted in a plurality of fixed-bed reactors, the plurality of fixed-bed reactors are connected in series and/or parallel.

Optionally, the reaction is conducted in an inactive atmosphere; and
the inactive atmosphere includes any one selected from the group consisting of nitrogen and an inert gas.

Specifically, the inert gas may be argon.

In the present application, the term "alkyl group" refers to a group obtained after any hydrogen atom is removed from an alkane compound, where the alkane compound includes a cycloalkane, a linear alkane, and a branched alkane; and
the "C₁-C₅" subscript indicates a number of carbon atoms in the group.

Possible beneficial effects of the present application:
1) The hydrolysis catalyst used in the present application is a molecular sieve catalyst with long life and high hydrolysis efficiency. The glycolic acid production method in the present application can be implemented by a traditional fixed-bed reactor under an atmospheric pressure, which is very suitable for continuous production. The raw material alkoxyacetate in the present application can be prepared by an environmentally-friendly and economical acetal carbonylation method.
2) When the raw material in the present application is methyl methoxyacetate, methanol and formaldehyde condensation to prepare DMM, DMM carbonylation to prepare methyl methoxyacetate, and methyl methoxyacetate hydrolysis to prepare glycolic acid can be used in combination to allow efficient, environmentally-friendly, and economical conversion of methanol (a platform chemical in the coal chemical industry) into glycolic acid.

### DETAILED DESCRIPTION OF THE EMBODIMENTS

The present application will be described in detail below with reference to embodiments, but the present application is not limited to these embodiments, but defined by the claims.

Unless otherwise specified, the raw materials in the embodiments of the present application all are purchased from commercial sources.

Possible embodiments are described below.

Specifically, the present application provides a method for preparing glycolic acid through hydrolysis of alkoxyacetate, where raw materials of alkoxyacetate and water are allowed to pass through a reaction zone loaded with an acidic molecular sieve catalyst, such that the raw materials undergo a reaction under specified conditions to produce glycolic acid.

The alkoxyacetate has a structural formula as follows: 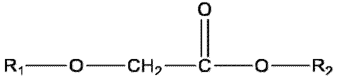 where R₁ is any one selected from the group consisting of methyl (CH₃), ethyl (C₂H₅), propyl (C₃H₇), and butyl (C₄H₉); R₂ is any one selected from the group consisting of methyl (CH₃), ethyl (C₂H₅), propyl (C₃H₇), and butyl (C₄H₉); and R₁ and R₂ can be the same or different.

The acidic molecular sieve is a molecular sieve with acidity.

The reaction zone includes a single fixed-bed reactor, or a plurality of fixed-bed reactors connected in series and/or parallel.

Conditions of the reaction are as follows: a reaction temperature is 60°C to 260°C, a molar ratio of the alkoxyacetate to the water is 1:20 to 20: 1, a reaction pressure is 0.1 MPa to 10 MPa, and a WHSV of the alkoxyacetate is 0.1 h⁻¹ to 3 h⁻¹.

A reaction equation for hydrolysis of the alkoxyacetate is as follows:

R₁OCH₂COOR₂ + 2 H₂O = R₁OH + R₂OH + HOCH₂COOH (1).

There are also two partial hydrolysis reactions for the alkoxyacetate as follows:

R₁OCH₂COOR₂ + H₂O = R₂OH + R₁OCH₂COOH (2)

and

R₁OCH₂COOR₂ + H₂O = R₁OH + HOCH₂COOR₂ (3).

Products of the two partial hydrolysis reactions can be further hydrolyzed under the same catalyst and reaction conditions to produce glycolic acid:

R₁OCH₂COOH + H₂O = R₁OH + HOCH₂COOH (4)

and

HOCH₂COOR₂+ H₂O = R₂OH + HOCH₂COOH (5).

In addition, alcohols R₁OH and R₂OH resulting from hydrolysis can also be partially dehydrated to produce corresponding ethers.

The acidic molecular sieve is one or a mixture of two or more selected from the group consisting of an acidic MFI-structured molecular sieve, an acidic FAU-structured molecular sieve, an acidic FER-structured molecular sieve, an acidic BEA-structured molecular sieve, an acidic MOR-structured molecular sieve, and an acidic MWW-structured molecular sieve.

The acidic molecular sieve is one or a mixture of two or more selected from the group consisting of an acidic ZSM-5 molecular sieve, an acidic Y molecular sieve, an acidic ZSM-35 molecular sieve, an acidic β molecular sieve, an acidic MOR molecular sieve, and an acidic MCM-22 molecular sieve.

The acidic molecular sieve is one or a mixture of two or more selected from the group consisting of a hydrogen-type ZSM-5 molecular sieve, a hydrogen-type Y molecular sieve, a hydrogen-type ZSM-35 molecular sieve, a hydrogen-type β molecular sieve, a hydrogen-type MOR molecular sieve, and a hydrogen-type MCM-22 molecular sieve.

A Si/Al atom ratio of the acidic molecular sieve is 3 to 500.

In addition to an acidic molecular sieve, the acidic molecular sieve catalyst further includes a catalyst forming agent; and the catalyst forming agent is one selected from the group consisting of alumina and silicon oxide, and has a weight percentage content of 0% to 50%.

The acidic molecular sieve catalyst is prepared by mixing the catalyst forming agent and the acidic molecular sieve and molding a resulting mixture into a strip.

In the alkoxyacetate, R₁ and R₂ both are methyl, that is, the alkoxyacetate is methyl methoxyacetate (CH₃OCH₂COOCH₃).

The methyl methoxyacetate is prepared by a DMM carbonylation method.

When the alkoxyacetate is methyl methoxyacetate, according to principles of reactions (1) to (5), hydrolysis products include glycolic acid, methoxyacetic acid (CH₃OCH₂COOH), methyl glycolate (HOCH₂COOCH₃), methanol, and dimethyl ether (DME). The methoxyacetic acid and methyl glycolate can be further hydrolyzed into glycolic acid, and methanol and DME can be returned to a DMM synthesis reactor to synthesize DMM.

When the alkoxyacetate is methyl methoxyacetate, according to the principles of reactions (1) to (5), the selectivity of the hydrolysis product glycolic acid can be as high as 50% based on a carbon number calculation theory.

The conditions of the reaction are preferably as follows: a reaction temperature is 130°C to 200°C, a molar ratio of the alkoxyacetate to the water is 1:8 to 1:2, a reaction pressure is 0.1 MPa to 0.3 MPa, and a WHSV of the alkoxyacetate is 0.3 h⁻¹ to 1 h⁻¹.

When the raw materials are allowed to pass through a reaction zone loaded with an acidic molecular sieve catalyst, an inert carrier gas selected from the group consisting of nitrogen and argon is introduced.

Analysis methods and conversion rate and selectivity calculation in the embodiments are as follows:
An Agilent7890B gas chromatograph is used to analyze products other than glycolic acid and unreacted raw materials, where an FID detector is connected to a DB-FFAP capillary column and a TCD detector is connected to a Porapak Q packed column. A liquid chromatograph is used to analyze glycolic acid, where a separation column is a C₁₈ column and a detector is an ultraviolet (UV) detector.

In an embodiment of the present application, both a conversion rate and selectivity are calculated based on a mole number of carbon: alkoxyacetate conversion rate = [(mole number of carbon in fed alkoxyacetate) - (mole number of carbon in discharge alkoxyacetate)] ÷ (mole number of carbon in fed alkoxyacetate) × 100% and selectivity for a product = (mole number of carbon in a discharged product) ÷ (total mole number of carbon in all discharge carbon-containing products × 100%.

The present application will be described in detail below with reference to examples, but the present application is not limited to these examples.

### ÷Catalyst performance test

### Example 1

An acidic H-ZSM-5 molecular sieve with a Si/Al ratio of 20 produced by Zhongke Catalysis New Technology (Dalian) Co., Ltd. was selected, crushed, and sieved to obtain 0.4 mm to 0.8 mm particles; 2 g of the particles was taken and filled into a stainless steel reaction tube with an inner diameter of 8 mm and activated with 50 mL/min nitrogen at 500°C for 4 h; a reaction was conducted for 24 h under the following conditions: a reaction temperature (T) was 150°C, and a reaction pressure (P) was 0.1 MPa; a molar ratio of methyl methoxyacetate to water was 1:4; and a WHSV of methyl methoxyacetate was 1.0 h⁻¹; and after the reaction was completed, products were analyzed by gas chromatography and liquid chromatography. Reaction results based on a mole number of carbon were shown in Table 1.

### Examples 2 to 9

The catalyst, reaction conditions, and reaction results were shown in Table 1. Other operations were the same as in Example 1.

**Table 1 Catalytic reaction results in Examples 1 to 9**

| **E xa m pl e** | **Acidic molecular sieve catalyst** | **Manufactur er** | **Si/Al ratio** | **Reaction conditions** | **Methyl methoxy acetate conversi on rate (%)** | **Glycoli c acid selectiv ity (%)** | **Methyl glycolat e selectivi ty (%)** | **Methox yacetic acid selectivi ty (%)** | **Methan ol and DME selectivi ty (%)** |
|---|---|---|---|---|---|---|---|---|---|
| 1 | H-ZSM-5 | Zhongke Catalysis New Technology (Dalian) Co., Ltd. | 20 | T = 150°C, P = 0.1 MPa, WHSV = 1.0 h⁻¹, and methyl methoxyacetate : water = 1:4 | 81.2 | 36.4 | 7.3 | 8.1 | 48.2 |
| 2 | H-Y | Zhongke Catalysis New Technology (Dalian) Co., Ltd. | 3 | T = 150°C, P = 0.1 MPa, WHSV = 1.0 h⁻¹, and methyl methoxyacetate : water = 1:4 | 60.3 | 30.4 | 14.4 | 100 | 45.2 |
| 3 | H-ZSM-35 | Catalyst Plant of Nankai University | 50 | T = 150°C, P = 0.1 MPa, WHSV = 1.0 h⁻¹, and methyl methoxyacetate : water = 1:4 | 78.9 | 37.0 | 7.5 | 7.0 | 48.5 |
| 4 | H-β | Zhongke Catalysis New Technology (Dalian) Co., Ltd. | 500 | T = 150°C, P = 0.1 MPa, WHSV = 1.0 h⁻¹, and methyl methoxyacetate : water = 1:4 | 40.3 | 26.2 | 10.7 | 20.0 | 43.1 |
| 5 | H-Mordenite | Yanchang Zhongke (Dalian) Energy Technology Co., Ltd. | 10 | T = 150°C, P = 0.1 MPa, WHSV = 1.0 h⁻¹, and methyl methoxyacetate : water = 1:4 | 50.7 | 29.0 | 14.0 | 12.5 | 44.5 |
| 6 | H-MCM-22 | Yanchang Zhongke (Dalian) Energy Technology Co., Ltd. | 100 | T = 150°C, P = 0.1 MPa, WHSV = 1.0 h⁻¹, and methyl methoxyacetate : water = 1:4 | 37.9 | 34.2 | 7.7 | 11.0 | 47.1 |
| 7 | H-ZSM-5 | Zhongke Catalysis New Technology (Dalian) Co., Ltd. | 20 | T = 260°C, P = 10 MPa, WHSV = 3.0 h⁻¹, and methyl methoxyacetate : water = 1:20 | 88.6 | 32.4 | 14.3 | 7.1 | 46.2 |
| 8 | H-ZSM-5 | Zhongke Catalysis New Technology (Dalian) Co., Ltd. | 20 | T = 60°C, P = 0.3 MPa, WHSV = 0.1 h⁻¹, and methyl methoxyacetate : water = 20:1 | 2.0 | 11.0 | 28.5 | 30.0 | 30.5 |
| 9 | H-ZSM-5 | Zhongke Catalysis New Technology (Dalian) Co., Ltd. | 20 | T = 150°C, P = 0.1 MPa, WHSV = 1.0 h⁻¹, methyl methoxyacetate : water = 1:4, and nitrogen = 50 mL/min | 77.6 | 36.0 | 8.9 | 7.1 | 48.0 |

It can be seen from Table 1 that the hydrogen-type molecular sieve catalyst leads to a high methyl methoxyacetate conversion rate and high glycolic acid selectivity during the hydrolysis of methyl methoxyacetate to produce glycolic acid, indicating excellent catalytic performance.

### Examples 10 to 13

Another alkoxyacetate was used instead of the methyl methoxyacetate in Example 1, and other conditions and operations remained unchanged. Reaction results were shown in Table 2.

**Table 2 Catalytic reaction results in Examples 1 and 10 to 13**

| **Example** | **Alkoxyacetate** | **Alkoxyacetate conversion rate (%)** | **Glycolic acid selectivity (%)** |
|---|---|---|---|
| 1 | Methyl methoxyacetate CH₃OCH₂COOCH₃ | 81.2 | 36.4 |
| 10 | Ethyl methoxyacetate CH₃OCH₂COOC₂H₅ | 80.5 | 28.1 |
| 11 | n-Propyl methoxyacetate CH₃OCH₂COOC₃H₇ | 83.9 | 25.1 |
| 12 | n-Butyl methoxyacetate CH₃OCH₂COOC₄H₉ | 78.8 | 25.3 |
| 13 | Ethyl ethoxyacetate C₂H₅OCH₂COOC₂H₅ | 79.1 | 31.1 |

It can be seen from Table 2 that the hydrogen-type molecular sieve catalyst can hydrolyze various types of alkoxyacetate into glycolic acid.

### Examples 14 to 15

The acidic H-ZSM-5 molecular sieve with a Si/Al ratio of 20 in Example 1 was molded with alumina or silicon oxide into a strip, and after the molding, a content of the alumina or silicon oxide in the molded catalyst was 20 wt%. Other conditions and operations remained unchanged. Reaction results were shown in Table 3.

**Table 3 Catalytic reaction results in Examples 1, 14, and 15**

| **Example** | **Forming agent** | **Methyl methoxyacetate conversion rate (%)** | **Glycolic acid selectivity (%)** |
|---|---|---|---|
| 1 | None | 81.2 | 36.4 |
| 14 | Alumina | 77.5 | 35.3 |
| 15 | Silicon oxide | 76.8 | 35.0 |

It can be seen from Table 3 that the catalytic activity of the acidic molecular sieve catalyst remains basically unchanged after the molding with alumina or silicon oxide.

### Example 16

An acidic H-ZSM-5 molecular sieve with a Si/Al ratio of 20 produced by Zhongke Catalysis New Technology (Dalian) Co., Ltd. was selected, crushed, and sieved to obtain 0.4 mm to 0.8 mm particles; 2 g of the particles was taken and filled into a stainless steel reaction tube with an inner diameter of 8 mm and activated with 50 mL/min nitrogen at 500°C for 4 h; a reaction was conducted under the following conditions: a reaction temperature (T) was 150°C, and a reaction pressure (P) was 0.1 MPa; a molar ratio of methyl methoxyacetate to water was 1:4; and a WHSV of methyl methoxyacetate was 1.0 h⁻¹; and products at different time points were analyzed by gas chromatography and liquid chromatography. Reaction results based on a mole number of carbon were shown in Table 4.

**Table 4 Catalytic reaction results in Example 16**

| **Reaction time (h)** | **Methyl methoxyacetate conversion rate (%)** | **Glycolic acid selectivity (%)** |
|---|---|---|
| 24 | 81.2 | 36.4 |
| 100 | 81.1 | 36.3 |
| 500 | 81.0 | 36.5 |
| 1000 | 80.6 | 36.1 |
| 2000 | 79.9 | 35.7 |
| 4000 | 76.6 | 35.2 |
| 8000 | 73.2 | 34.8 |

It can be seen from Table 4 that the acidic molecular sieve catalyst exhibits excellent stability in the hydrolysis of methyl methoxyacetate to produce glycolic acid, and can meet the requirements of industrial use.

The above examples are merely few examples of the present application, and do not limit the present application in any form. Although the present application is disclosed as above with preferred examples, the present application is not limited thereto.

## Claims

1. A method for preparing glycolic acid through a hydrolysis of alkoxyacetate, comprising: subjecting raw materials comprising the alkoxyacetate and water to a reaction in the presence of an acidic molecular sieve catalyst to produce the glycolic acid,
wherein the alkoxyacetate is at least one selected from the group consisting of compounds with a structural formula shown in formula I: 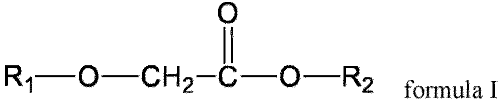
wherein R₁ and R₂ each are independently one selected from the group consisting of C₁-C₅ alkyl groups.

2. The method according to claim 1, wherein R₁ is one selected from the group consisting of methyl, ethyl, propyl, and butyl; and
R₂ is one selected from the group consisting of methyl, ethyl, propyl, and butyl.

3. The method according to claim 1, wherein the acidic molecular sieve catalyst comprises an acidic molecular sieve.

4. The method according to claim 3, wherein the acidic molecular sieve is at least one selected from the group consisting of an acidic MFI-structured molecular sieve, an acidic FAU-structured molecular sieve, an acidic FER-structured molecular sieve, an acidic BEA-structured molecular sieve, an acidic mordenite (MOR)-structured molecular sieve, and an acidic MWW-structured molecular sieve.

5. The method according to claim 4, wherein the acidic molecular sieve is at least one selected from the group consisting of an acidic ZSM-5 molecular sieve, an acidic Y molecular sieve, an acidic ZSM-35 molecular sieve, an acidic β molecular sieve, an acidic MOR molecular sieve, and an acidic MCM-22 molecular sieve.

6. The method according to claim 5, wherein the acidic molecular sieve is at least one selected from the group consisting of a hydrogen-type ZSM-5 molecular sieve, a hydrogen-type Y molecular sieve, a hydrogen-type ZSM-35 molecular sieve, a hydrogen-type β molecular sieve, a hydrogen-type MOR molecular sieve, and a hydrogen-type MCM-22 molecular sieve.

7. The method according to claim 3, wherein a Si/Al atom ratio of the acidic molecular sieve is 3 to 500.

8. The method according to claim 3, wherein the acidic molecular sieve catalyst further comprises a forming agent;
the forming agent is an oxide;
the oxide is at least one selected from the group consisting of alumina and silicon oxide; and
a content of the forming agent in the acidic molecular sieve catalyst is m, and 0 < m ≤ 50 wt%.

9. The method according to claim 1, wherein conditions of the reaction are as follows:
a reaction temperature is 60°C to 260°C;
a reaction pressure is 0.1 MPa to 10 MPa;
a molar ratio of the alkoxyacetate to the water is 1:20 to 20:1; and
a weight hourly space velocity (WHSV) of the alkoxyacetate is 0.1 h⁻¹ to 3 h⁻¹.

10. The method according to claim 9, wherein the conditions of the reaction are as follows:
the reaction temperature is 130°C to 260°C;
the reaction pressure is 0.1 MPa to 0.3 MPa;
the molar ratio of the alkoxyacetate to the water is 1:2 to 1:8; and
the WHSV of the alkoxyacetate is 0.3 h⁻¹ to 1 h⁻¹.

11. The method according to claim 1, wherein the reaction is conducted in one fixed-bed reactor or a plurality of fixed-bed reactors.

12. The method according to claim 11, wherein the plurality of fixed-bed reactors are connected in series and/or parallel.

13. The method according to claim 1, wherein the reaction is conducted in an inactive atmosphere; and
the inactive atmosphere comprises one selected from the group consisting of nitrogen and an inert gas.

## Patentansprüche

1. Ein Verfahren zur Herstellung von Gylkolsäure durch eine Hydrolyse von Alkoxyacetat, umfassend: Unterziehen von Rohmaterialien, umfassend das Alkoxyacetat und Wasser, einer Reaktion in Gegenwart eines sauren Molekularsiebkatalysators, um die Glykolsäure herzustellen,
wobei das Alkoxyacetat mindestens eines ist, das aus der Gruppe ausgewählt ist, die aus Verbindungen mit einer in Formel I gezeigten Strukturformel besteht: 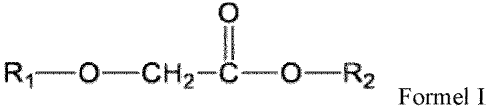
wobei R₁ und R₂ jeweils unabhängig voneinander aus der Gruppe ausgewählt sind, die aus C₁-C₅-Alkylgruppen besteht.

2. Verfahren nach Anspruch 1, wobei R₁ aus der Gruppe ausgewählt ist, die aus Methyl, Ethyl, Propyl und Butyl besteht; und
R₂ aus der Gruppe ausgewählt ist, die aus Methyl, Ethyl, Propyl und Butyl besteht.

3. Verfahren nach Anspruch 1, wobei der saure Molekularsiebs-Katatysator ein saures Molekularsieb umfasst.

4. Verfahren nach Anspruch 3, wobei das saure Molekularsieb mindestens eines ist, das ausgewählt ist aus der Gruppe, bestehend aus einem sauren MFI-strukturierten Molekularsieb, einem sauren FAU-strukturierten Molekularsieb, einem sauren FER-strukturierten Molekularsieb, einem sauren BEA-strukturierten Molekularsieb, einem sauren Mordenit (MOR)-strukturierten Molekularsieb und einem sauren MWW-strukturierten Molekularsieb.

5. Verfahren nach Anspruch 4, wobei das saure Molekularsieb mindestens eines ist, das ausgewählt ist aus der Gruppe, bestehend aus einem sauren ZSM-5-Molekularsieb, einem sauren Y-Molekularsieb, einem sauren ZSM-35-Molekularsieb, einem sauren β-Molekularsieb, einem sauren MOR-Molekularsieb und einem sauren MCM-22-Molekularsieb.

6. Verfahren nach Anspruch 5, wobei das saure Molekularsieb mindestens eines ist, das ausgewählt ist aus der Gruppe, bestehend aus einem ZSM-5-Molekularsieb vom Wasserstofftyp, einem Y-Molekularsieb vom Wasserstofftyp, einem ZSM-35-Molekularsieb vom Wasserstofftyp, einem β-Molekularsieb vom Wasserstofftyp, einem MOR-Molekularsieb vom Wasserstofftyp und einem MCM-22-Molekularsieb vom Wasserstofftyp.

7. Verfahren nach Anspruch 3, wobei das Si/Al-Atomverhältnis des sauren Molekularsiebs 3 bis 500 beträgt.

8. Verfahren nach Anspruch 3, wobei der saure Molekularsiebs-Katatysator ferner ein Formungsmittel umfasst;
das Formungsmittel ein Oxid ist;
das Oxid mindestens eines ist, das ausgewählt ist aus der Gruppe, die aus Aluminiumoxid und Siliciumoxid besteht; und
der Gehalt des Formungsmittels in dem sauren Molekularsiebs-Katatysator m beträgt und 0 < m ≤ 50 Gew.-% ist.

9. Verfahren nach Anspruch 1, wobei die Reaktionsbedingungen wie folgt sind:
eine Reaktionstemperatur beträgt 60 °C bis 260 °C;
ein Reaktionsdruck beträgt 0,1 MPa bis 10 MPa;
das Molverhältnis von Alkoxyacetat zu Wasser 1:20 bis 20:1 beträgt und
die stündliche Gewichts-Raum-Geschwindigkeit (weight hourly space velocity, WHSV) des Alkoxyacetats 0,1 h⁻¹ to 3 h⁻¹ beträgt.

10. Verfahren nach Anspruch 9, wobei die Reaktionsbedingungen wie folgt sind: die Reaktionstemperatur beträgt 130 °C bis 260 °C;
der Reaktionsdruck 0,1 MPa bis 0,3 MPa beträgt;
das Molverhältnis von Alkoxyacetat zu Wasser 1:2 bis 1:8 beträgt; und
die WHSV des Alkoxyacetats 0,3 h⁻¹ bis 1 h⁻¹ beträgt.

11. Verfahren nach Anspruch 1, wobei die Reaktion in einem Festbettreaktor oder mehreren Festbettreaktoren durchgeführt wird.

12. Verfahren nach Anspruch 11, wobei die mehreren Festbettreaktoren in Reihe und/oder parallel geschaltet sind.

13. Verfahren nach Anspruch 1, wobei die Reaktion unter inaktiver Atmosphäre durchgeführt wird; und
die inaktive Atmosphäre eines umfasst, das ausgewählt ist aus der Gruppe, die aus Stickstoff und einem Inertgas besteht.

## Revendications

1. Procédé de préparation d'acide glycolique par hydrolyse d'alcoxyacétate, comprenant : la soumission de matières premières comprenant l'alcoxyacétate et de l'eau à une réaction en présence d'un catalyseur de tamis moléculaire acide pour produire l'acide glycolique,
dans lequel l'alcoxyacétate est au moins l'un parmi les composés ayant une formule structurale représentée dans la formule I : 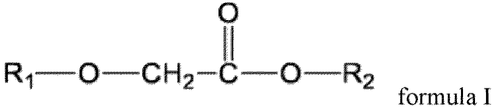
dans laquelle R₁ et R₂ sont chacun indépendamment l'un quelconque parmi les groupes alkyle en C₁-C₅.

2. Procédé selon la revendication 1, dans lequel R₁ est l'un quelconque parmi le méthyle, l'éthyle, le propyle et le butyle; et
R₂ est l'un quelconque parmi le méthyle, l'éthyle, le propyle et le butyle.

3. Procédé selon la revendication 1, dans lequel le catalyseur de tamis moléculaire acide comprend un tamis moléculaire acide.

4. Procédé selon la revendication 3, dans lequel le tamis moléculaire acide est au moins l'un parmi un tamis moléculaire à structure MFI acide, un tamis moléculaire à structure FAU acide, un tamis moléculaire à structure FER acide, un tamis moléculaire à structure BEA acide, un tamis moléculaire à structure mordénite (MOR) acide et un tamis moléculaire à structure MWW acide.

5. Procédé selon la revendication 4, dans lequel le tamis moléculaire acide est au moins l'un parmi un tamis moléculaire ZSM-5 acide, un tamis moléculaire Y acide, un tamis moléculaire ZSM-35 acide, un tamis moléculaire β acide, un tamis moléculaire MOR acide et un tamis moléculaire MCM-22 acide.

6. Procédé selon la revendication 5, dans lequel le tamis moléculaire acide est au moins l'un parmi un tamis moléculaire ZSM-5 de type hydrogène, un tamis moléculaire Y de type hydrogène, un tamis moléculaire ZSM-35 de type hydrogène, un tamis moléculaire β de type hydrogène, un tamis moléculaire MOR de type hydrogène, et un tamis moléculaire MCM-22 de type hydrogène.

7. Procédé selon la revendication 3, dans lequel un rapport atomique Si/Al du tamis moléculaire acide est de 3 à 500.

8. Procédé selon la revendication 3, dans lequel le catalyseur de tamis moléculaire acide comprend en outre un agent formant ;
l'agent formant est un oxyde ;
l'oxyde est au moins l'un parmi l'alumine et l'oxyde de silicium ; et
une teneur de l'agent formant dans le catalyseur de tamis moléculaire acide est m, et 0 < m ≤ 50 % en poids.

9. Procédé selon la revendication 1, dans lequel les conditions de la réaction sont comme suit :
une température de réaction de 60°C à 260°C ;
une pression de réaction de 0,1 MPa à 10 MPa ;
un rapport molaire de l'alcoxyacétate à l'eau de 1:20 à 20:1 ; et
une vitesse spatiale horaire pondérale (weight hourly space velocity, WHSV) de l'alcoxyacétate de 0,1 h⁻¹ à 3 h⁻¹.

10. Procédé selon la revendication 9, dans lequel les conditions de la réaction sont comme suit : la température de réaction est de 130°C à 260°C ;
la pression de réaction est de 0,1 MPa à 0,3 MPa ;
le rapport molaire de l'alcoxyacétate à l'eau est de 1:2 à 1:8 ; et
la WHSV de l'alcoxyacétate est de 0,3 h⁻¹ à 1 h⁻¹.

11. Procédé selon la revendication 1, dans lequel la réaction est conduite dans un réacteur à lit fixe ou une pluralité de réacteurs à lit fixe.

12. Procédé selon la revendication 11, dans lequel la pluralité de réacteurs à lit fixe sont raccordés en série et/ou en parallèle.

13. Procédé selon la revendication 1, dans lequel la réaction est conduite dans une atmosphère inactive ; et
l'atmosphère inactive comprend l'un quelconque parmi l'azote et un gaz inerte.
